(19) **Europäisches Patentamt / European Patent Office / Office européen des brevets**

(11) **EP 3 311 737 B1**

(12) # EUROPEAN PATENT SPECIFICATION

(45) Date of publication and mention
of the grant of the patent:
**01.04.2020 Bulletin 2020/14**

(21) Application number: **15832670.2**

(22) Date of filing: **22.09.2015**

(51) Int Cl.:
**A61B 5/0245** *(2006.01)*     **A61B 5/021** *(2006.01)*

(86) International application number:
**PCT/CN2015/090278**

(87) International publication number:
**WO 2016/201817 (22.12.2016 Gazette 2016/51)**

(54) **DEVICE AND METHOD FOR DETECTING PULSE CYCLE, AND WEARABLE ELECTRONIC DEVICE**

VORRICHTUNG UND VERFAHREN ZUR ERKENNUNG EINES PULSZYKLUS UND AM KÖRPER TRAGBARE ELEKTRONISCHE VORRICHTUNG

DISPOSITIF ET PROCÉDÉ DE DÉTECTION DE CYCLE D'IMPULSION, ET DISPOSITIF ÉLECTRONIQUE POUVANT ÊTRE PORTÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2015 CN 201510343249**

(43) Date of publication of application:
**25.04.2018 Bulletin 2018/17**

(73) Proprietor: **BOE Technology Group Co., Ltd.
Beijing 100015 (CN)**

(72) Inventor: **YUAN, Zuo
Beijing 100176 (CN)**

(74) Representative: **Fritzsche, Thomas
Fritzsche Patent
Naupliastraße 110
81545 München (DE)**

(56) References cited:

| | |
|---|---|
| CN-A- 1 537 508 | CN-A- 101 703 396 |
| CN-A- 102 307 520 | US-A1- 2003 236 647 |
| US-A1- 2012 197 140 | US-A1- 2014 114 580 |
| US-A1- 2014 243 628 | US-B1- 6 575 912 |

- YU, WEI: 'Experimental Study on Non-invasive Reflectance Oxygen Saturation Measurement, CNKI' MEDICINE AND HEALTH 15 January 2007, pages 13, 14, 38 - 46, XP009500364
- ZHANG, XIN ET AL.: 'Improvement of Pulse Waveform Dectection with Autoregressive Model In Blood Oxygen Saturation Measurement' JOURNAL OF BIOMEDICAL ENGINEERING vol. 17, no. 3, 31 December 2000, XP009500226

**Description**

TECHNICAL FIELD

The disclosure relates to the field of vital sign detection technologies, and in particular, to a pulse cycle detection device and method, and a wearable electronic device.

BACKGROUND

Recently, wearable health monitoring devices have received increased attention. Among common types of wearable pulse (heart rate) monitoring devices is a blood oxygen sensor based device. The principle of pulse measurement is based on the following fact: absorption of light by oxyhemoglobins and reduced hemoglobins in blood changes with periodic variation of pulse waves, and pulse detection may be achieved by detecting the change of amount of absorption of light by the blood. In other words, the pulse wave signal is modulated in the measured light signal. The pulse wave cycle or heart rate (the pulse frequency may be considered as the heart rate) may be computed by analyzing a photoplethysmography (PPG) signal detected by the blood oxygen sensor.

Depending on its acquisition mode, the blood oxygen sensor may be a transmission-type sensor or a reflection-type sensor. Since the reflection-type sensor may be suitable for measuring various parts such as the arm or wrist, forehead, earlobe, and will not induce discomfort (for example, oppression induced by a transmission-type sensor based finger clip device) of a subject, it has a good application prospect in wearable devices. However, the PPG signal detected by the reflection-type sensor is generally weaker than the transmission-type sensor, such that its measurement accuracy is affected.

In addition, a differential threshold method is one of common pulse wave signal analysis methods, by which a peak-to-peak value of a pulse wave signal may be easily determined. A typical differential threshold method is based on the following idea.

Assume that $S_1$ is a (preprocessed) pulse wave signal sequence, $S_2$ is calculated as follows:

$$S_2(i) = \begin{cases} 0 & \text{if } S_1(i) \leq S_1(i-k) \\ S_1(i) - S_1(i-k) & \text{if } S_1(i) > S_1(i-k) \end{cases}$$

where $S_2$ is a special differentiation of the pulse wave signal $S_1$, and $k$ is a step, which is generally an empirical value. For example, when the sampling frequency is 100Hz, $k$ may take a value of 5 to 10.

Based on the differential value sequence $S_2$, it yields

$$S_3(i) = \begin{cases} 0 & \text{if } S_2(i) \leq S_{2\max}/2 \\ S_2(i) - S_{2\max}/2 & \text{if } S_2(i) > S_{2\max}/2 \end{cases},$$

where the threshold $S_{2\max}$ is the maximum of $S_2(i\text{-}1)$, $S_2(i\text{-}2)$, ..., $S_2(i\text{-}n)$. The pulse cycle may be determined from the waveform of $S_3$.

The above method requires recording a section of pulse wave signal $S_1$, and deriving $S_3$ after obtaining the differential value sequence $S_2$ of the pulse wave signal $S_1$, and further calculating the pulse cycle from the waveform of $S_3$. Therefore, the traditional differential threshold method is computationally intensive and has difficulty in meeting requirements of a real-time application. In addition, since the parts for pulse waves detection are generally located in a peripheral blood circulation, where blood vessels have a strong smoothing effect on pulse waves, particularly when the pulsation of the subject is weak, the amplitude and the steepness of rising edges of the pulse waves are significantly reduced, such that the anti-interference ability and the measurement accuracy of the traditional differential threshold method become very poor, resulting in frequent occurrence of false detections or missed detections.

Thus, there is a need for an improved pulse cycle detection device and method.

US2014/243628-A1, US2003/236647-A1, US2014/114580-A1, US6-575-912-B1, and US6-575-912-B1 show different methods for pulse cycle detection.

SUMMARY

It is advantageous to achieve a pulse cycle detection device and method which may solve at least one of the above-mentioned problems.

In a first aspect of the disclosure, a pulse cycle detection device is provided comprising: a sensor for sensing a pulse wave signal of a subject; and a processor for processing a digital pulse wave signal which is analog-to-digital converted from the pulse wave signal sensed by the sensor, to detect the pulse cycle of the subject. The processor is configured to perform operations of: calculating a differential value of the digital pulse wave signal over time; determining that the pulse wave signal reaches a maximum point and recording a time value corresponding to the maximum point, whenever a transition from positive to negative occurs to the differential value; recognizing a last recorded maximum point as a peak point of the pulse wave signal, once the differential value is less than a dynamic threshold; and calculating a difference between time values corresponding to two successive peak points for deriving the pulse cycle.

According to a second aspect of the disclosure, a pulse cycle detection method is provided for processing a digital pulse wave signal to detect the pulse cycle of a subject, comprising: calculating a differential value of the digital pulse wave signal over time; determining that the pulse wave signal reaches a maximum point and recording a time value corresponding to the maximum point, whenever a transition from positive to negative occurs to the differential value; recognizing a last recorded maximum point as a peak point of the pulse wave signal, once the differential value is less than a dynamic threshold; and calculating a difference between time values corresponding to two successive peak points for deriving the pulse cycle.

According to a third aspect of the disclosure, a wearable electronic device is provided comprising a pulse cycle detection device provided by the first aspect of the disclosure.

These and other aspects of the disclosure will be apparent from and elucidated with reference to embodiments described below.

BRIEF DESCRIPTION OF THE DRAWINGS

Fig. 1 illustrates schematically a block diagram of a pulse cycle detection device according to an embodiment of the disclosure;
Fig. 2(a) illustrates schematically a light path of a pulse cycle detection device which employs a reflection-type blood oxygen sensor when in operation according to an embodiment of the disclosure;
Fig. 2(b) illustrates schematically a plan view of a structure of a reflection-type blood oxygen sensor in a pulse cycle detection device according to an embodiment of the disclosure;
Fig. 2(c) illustrates schematically a plan view of another structure of a reflection-type blood oxygen sensor in a pulse cycle detection device according to an embodiment of the disclosure;
Fig. 3 illustrates a flow chart of a pulse cycle detection method according to an embodiment of the disclosure;
Fig. 4 illustrates a waveform of an exemplary pulse wave signal and a corresponding differential value sequence;
Fig. 5 illustrates operations in a step of calculating the pulse cycle in the method as shown in Fig. 3; and
Fig. 6 illustrates operations in a step of calculating the heart rate in the method as shown in Fig. 3.

DETAILED DESCRIPTION

Embodiments of the disclosure will be described in detail below in connection with the drawings.

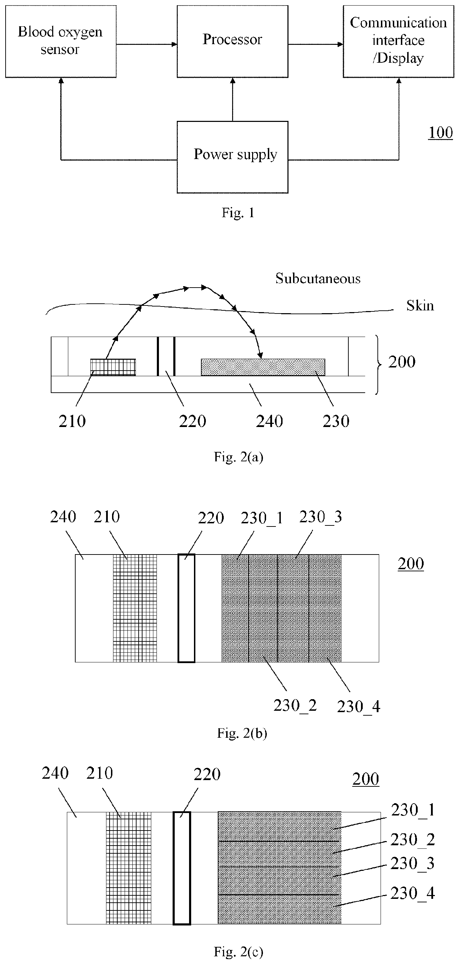

Fig. 1 illustrates schematically a block diagram of a pulse cycle detection device 100 according to an embodiment of the disclosure. The pulse cycle detection device 100 may comprise a sensor, a processor, a power supply and a communication interface and/or a display. As shown, the sensor, the processor and the communication interface and/or the display are successively electrically connected, and the power supply is electrically connected with individual components. The sensor is used for sensing a pulse wave signal of a subject. In particular, the sensor may be a transmission-type blood oxygen sensor or a reflection-type blood oxygen sensor. The processor may be used for processing a digital pulse wave signal which is analog-to-digital converted from the pulse wave signal sensed by the sensor, to detect the pulse cycle of the subject. The processor may further be used for calculating a heart rate based on the pulse cycle of the subject. In a wearable application, an ARM core based processor may, for example, be used. The display may be used for displaying at least one of the digital pulse wave signal, the pulse cycle or the heart rate. The communication interface may be used for sending at least one of the digital pulse wave signal, the pulse cycle and the heart rate to another receiving device, such as a smart terminal, for display and storage. The communication interface may be a wireless interface, such as infrared, Bluetooth, or Wi-Fi, or a wired interface, such as a serial interface, a universal serial bus (USB), or I2C. The power supply is used for powering the individual components of the detection device 100. A lithium battery may be used as the power supply, for example.

Fig. 2(a) illustrates schematically a light path of a pulse cycle detection device which employs a reflection-type blood oxygen sensor 200 when in operation according to an embodiment of the disclosure. As shown, in the case of a reflection-type sensor 200, a light source (LED 210) and a photosensitive receiving device (a photodiode 230) are arranged on one and the same side of a substrate 240. Between the LED 210 and the photodiode 230 is disposed an occluder 220. The occluder 220 is used for occluding light emitted from the LED 210, such that it will not directly illuminate the photodiode 230. The incident light generated by the LED light source 210 is scattered by the subcutaneous tissue of the subject for several times, and a part of the light returns to the skin surface again. The light signal reflected back by the tissue of the subject is received by the photodiode 230 and converted to an electrical signal.

In an embodiment, the pulse cycle detection device 100 may comprise an isolation cushion (not shown in the figure), which is disposed surrounding the reflection-type blood oxygen sensor 200 and may function to isolate ambient light, thereby reducing the interference of ambient illumination conditions to the reflection-type blood oxygen sensor 200. This is conducive to further improve the detection accuracy. Depending on the form design of the pulse cycle detection device 100, the isolation cushion may be of a hollow square shape, or an annular shape (not shown). In addition, in a wearable application, the isolation cushion may further improve the comfort of a user when wearing the wearable device. For example, the isolation cushion may contact with human skin, and at the same time surround the reflection-type blood oxygen sensor 200. This way, the comfort of the wearer is improved and the detection accuracy is increased.

Fig. 2(b) illustrates schematically a plan view of a structure of the reflection-type blood oxygen sensor 200 in a pulse cycle detection device according to an embodiment of the disclosure. In the reflection-type blood oxygen sensor 200, the photodiode 230 may comprise multiple photodiode units 230_1, 230_2, 230_3, 230_4 (4 photodiode units are shown by way of example in the figure) integrated together. In this figure, the integrated photodiode units 230_1, 230_2, 230_3 and 230_4 are illustrated as being arranged side by side on the substrate 240 in a way in which their distances to the LED 210 increase progressively.

Fig. 2(c) illustrates schematically a plan view of another structure of the reflection-type blood oxygen sensor 200 in a pulse cycle detection device according to an embodiment of the disclosure, wherein the integrated photodiode units 230_1, 230_2, 230_3 and 230_4 are illustrated as being arranged side by side on the substrate 240 in a way in which their distances to the LED 210 are equal.

In either case, since the photodiode units 230_1, 230_2, 230_3 and 230_4 are integrated together, the distance therebetween may be ignored. They may receive a light signal reflected from substantially the same subcutaneous tissue of the subject. Thus, an individual photodiode unit 230_x (x=1, 2, 3, 4...) constitutes a separate signal channel. Each signal channel may provide the processor with a separate pulse wave signal. In subsequent operations performed by the processor (discussed below), data of multiple channels may be utilized (for example, an average value of the data of the multiple channels may be taken) to improve the detection accuracy of the pulse cycle. Additionally, in an application of an embodiment of the disclosure, preferably, a LED device emitting green light (e.g., at wavelength of 500~560nm) is used as the LED 210. The green light may provide good penetrability, and therefore provide a pulse wave signal with a good signal intensity and signal-to-noise ratio.

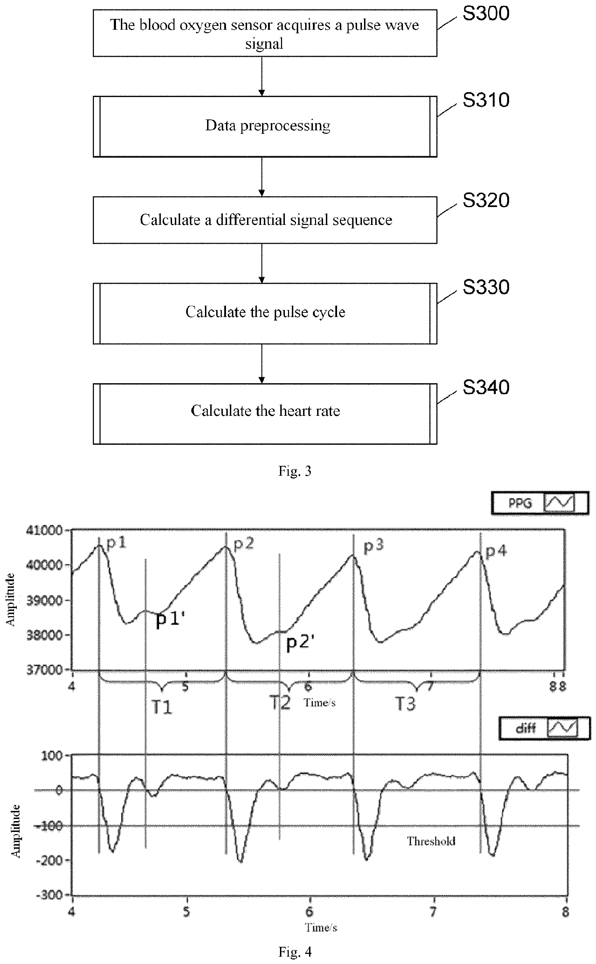

Fig. 3 illustrates a flow chart of a pulse cycle detection method according to an embodiment of the disclosure.

At step S300, a pulse wave signal of a subject is sensed by the blood oxygen sensor. As described previously, the blood oxygen sensor may be a transmission-type sensor or a reflection-type sensor. In an embodiment, a reflection-type sensor with integrated multiple photodiode units 230_x as described previously may be employed as the blood oxygen sensor.

At step S310, the pulse wave signal sensed by the blood oxygen sensor is preprocessed by the processor. The preprocessing may comprise at least one of moving average filtering and band-pass filtering. The moving average filtering is for example 10-point moving average filtering. By way of the moving average filtering, mutational components in the pulse wave signal may be filtered out. The band-pass filtering is for example accomplished by a band-pass filter with a pass band of 0.1Hz~10Hz to reduce noise interference. In the case of the integrated reflection-type sensor as described previously, the preprocessing may further comprise first taking an average value of the data of multiple channels. By taking the average value of the data of multiple channels, the sampling accuracy of the pulse wave signal may be improved, and hence the accuracy of the detected pulse cycle. It should further be appreciated that an analog signal needs to be converted to a digital signal in the sampling procedure. The analog-digital conversion may for example be accomplished by an A/D converter embedded in the blood oxygen sensor. Alternatively, the analog-digital conversion may be accomplished by an A/D converter embedded in a processor chip or an A/D converter separate from the processor chip.

At step S320, a differential value of the analog-to-digital converted digital pulse wave signal is calculated by the processor in real time. Assume that $S_1$ is a (preprocessed) pulse wave signal sequence, then the differential value sequence $S_2$ is calculated as follows

$$S_2(i) = \begin{cases} 0 & \text{if } S_1(i) \leq S_1(i-k) \\ S_1(i) - S_1(i-k) & \text{if } S_1(i) > S_1(i-k) \end{cases}$$

where *k* is a step.

In an embodiment, the differential value of the digital pulse wave signal may be calculated point-by-point, namely, the step *k*=1. This way, the detection accuracy may be improved at a cost of an amount of calculation.

At step S330, the pulse cycle is calculated by the processor from the characteristics of the differential value sequence diff of the pulse wave signal (discussed later).

Optionally, at step S340, the processor further calculates the heart rate from the calculated pulse cycle.

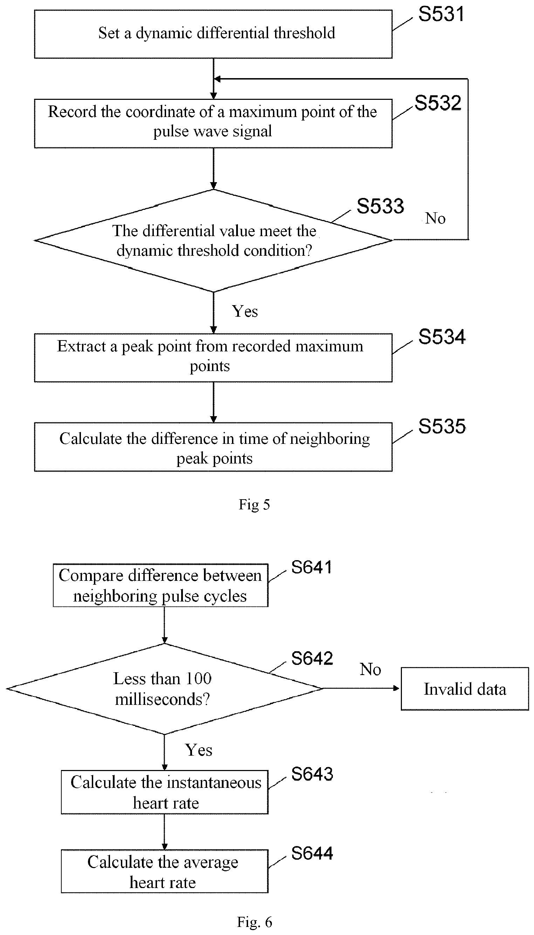

The flow of the step S330 will be described in detail below in connection with Fig. 4 and Fig. 5. Fig. 4 illustrates a waveform of an exemplary pulse wave signal (PPG) and the corresponding differential value sequence (diff), and Fig. 5 illustrates operations in the step S330 of calculating the pulse cycle in the method as shown in Fig. 3.

In this embodiment, the pulse cycle is calculated based on an improved differential threshold method in which the pulse cycle is directly calculated utilizing the differential value without the need for further processing of the differential value of the pulse wave signal, including taking the differential value of the differential value sequence (a second order derivative), shifting down the differential value, or the like.

As shown in Fig. 5, at step S531, a dynamic differential threshold is set. The so-called "dynamic" means that the threshold changes over time (discussed later).

At step S532, the time value when the pulse wave signal arrives at a maximum point is recorded. Since the maximum point of the pulse wave signal corresponds to a transition of the differential value from positive to negative (a zero-crossing), it may be determined whether the pulse wave signal reaches a maximum point by detecting the transition of the differential value. If the transition of the differential value from positive to negative is detected, the corresponding time value is recorded. In three cycles T1, T2 and T3 of the exemplary pulse wave signal as shown in Fig. 4, 6 maximum points may be detected, including 4 real peak points p1, p2, p3 and p4 and 2 local maximum points p1' and p2'. In the detection procedure, the dynamic threshold mentioned above is utilized to recognize which maximum points are real peak points.

At step S533, it is judged whether a current differential value meets the dynamic threshold condition. The relationship between the differential value and the dynamic threshold in fact reflects information on steep falling edges of the pulse wave signal. It may be seen from Fig. 4 that during the steep falling edges of the pulse wave signal PPG, the peak points p1, p2, p3 and p4 (which correspond to the transition of the differential value from positive to negative) of the pulse wave signal PPG has a temporal relevance to minimums of the differential value sequence diff. That is, for each of the peak points p1, p2, p3 and p4, after the transition of the differential value from positive to negative, is followed a minimum of the differential value, whereas for the local maximum points p1' and p2', it is not the case. Therefore, such time relevance may be utilized to recognize real peak points.

At step S534, specifically, once the current differential value is less than the dynamic threshold, the last maximum point recorded in an elapsed time is recognized as a peak point of the pulse wave signal. For example, in the example waveform as shown in Fig. 4, after the maximum point p1 of the pulse wave signal is determined, once it is detected that the differential value is less than the dynamic threshold, the last maximum point (i.e., p1) recorded in the elapsed time is recognized as a peak point. As such, a peak point may be determined in tens of milliseconds (a period of time in which the differential value transits from positive to negative and to further become less than the dynamic threshold, as shown in Fig. 4).

In an embodiment, the dynamic threshold may be 1/2 of the minimum differential value in an elapsed predetermined time interval. For example, the predetermined time interval is 4 seconds. Each time a differential value is obtained by calculation, it is judged whether the differential value is less than the dynamic threshold, and if yes, the recognition of a peak point is triggered. In such a case, the dynamic threshold needs to be updated in each judgment, and searching for a minimum differential value (for example, using a known search algorithm in the art) in the elapsed 4 seconds from the current moment is involved. Thus, the searching and updating operation may incur a heavy computational burden, especially in the case of calculating the differential value point by point.

In another embodiment, the dynamic threshold may be 1/2 of the minimum differential value in the previous threshold update cycle. The scheme of the threshold update cycle is like this: the threshold is updated once every a fixed period of time from the moment at which the detection is started. For example, a variable representative of the minimum differential value may be maintained for the threshold update cycle, and in each threshold update cycle, each time a differential value is obtained by calculation, it is compared with the variable. If the calculated differential value is less than the value of the variable, the calculated differential value is utilized to update the variable. On entering a new threshold update cycle, the threshold updated in the previous threshold update cycle is used as the threshold to be used in the current threshold update cycle. In the example in Fig. 4, the threshold update cycle is set to be 4 seconds, and therefore in the period of time of 4 seconds as shown in the figure, the dynamic threshold is kept constant. Thus, frequent update of the threshold may be avoided, thereby reducing the computational burden.

Since the two dynamic thresholds discussed above are associated with a particular duration, they may not be adapted to a drastic change in the pulse cycle (for example, in a situation where the subject changes from a calm state to a motion state).

In yet another embodiment, the dynamic threshold may be 1/2 of the minimum differential value in a predetermined number of elapsed pulse cycles. Such a dynamic threshold is associated with several recent pulse cycles in the elapsed time, but not a specific duration, and therefore it can follow the drastic change in the pulse cycle.

It should be further appreciated that since the initial value of the dynamic threshold is generally preset to be zero, there will be a period of preparation time in an initial detection, during which the detection result of the pulse cycle is wrong because the correct dynamic threshold has not yet been established. Advantageously, for dynamic thresholds as mentioned previously, the predetermined time interval or the threshold update cycle may be set to be less than or equal to 2 seconds and greater than or equal to 1 second, or the predetermined number may for example be 1, 2 or 3. In an alterative embodiment, the predetermined time interval or the threshold update cycle may be set to be less than or equal to 2 seconds and greater than or equal to 1 second in a predetermined period of time from the moment at which the detection starts, and set to be 4 seconds after the predetermined period of time. During this predetermined period of time, the correct dynamic threshold is established. The predetermined period of time may be 2 seconds, 3 seconds or even more. Thus, both the fast commissioning of the detection device after being turned on and the stability of the detection procedure are considered.

At step S535, a difference in time between two neighboring peak points is calculated to derive the pulse cycle. In an embodiment, the difference in time between two successive peak points may be calculated after multiple peak points have been recognized. This is advantageous to a situation where an average value of multiple pulse cycles needs to be calculated to improve the detection accuracy. However, since it has to wait for recording of multiple peak points, such a scheme suffers from loss in terms of real time. In an alternative embodiment, the difference between time values corresponding to two successive peak points may be calculated immediately whenever these two peak points are recognized. As described previously, since a peak point may be determined in tens of milliseconds, after a pulse cycle is finished, the pulse cycle (heart rate) can be calculated in tens of milliseconds at the soonest. This greatly enhances the timeliness of the pulse cycle detection.

Fig. 6 illustrates operations in a step of calculating the heart rate in the method as shown in Fig. 3.

At step S641, the difference in time between recently measured two pulse cycles (namely, the currently measured pulse cycle and the last measured pulse cycle) is compared. At step S642, it is judged whether the difference in time is less than a predetermined threshold, and if yes, it is determined that the currently measured pulse cycle is valid; otherwise, it is determined that the currently measured pulse cycle is invalid. Medical researches show that under normal circumstances, there may be a difference between two neighboring pulse cycles, which may be up to tens of milliseconds (for human being). If the difference exceeds a predetermined threshold (e.g., 100 milliseconds), it may be inferred that the measured pulse cycle suffers from interference and thereby is invalid. If the difference does not exceed the predetermined threshold, at step S643, the instantaneous heart rate is calculated from the valid pulse cycle. For example, if the measured valid pulse cycle is 0.8 seconds, the heart rate is 60/0.8 = 75 times/minute. Optionally, at step S644, in a non-time-critical application, the average value of multiple (e.g., 5) heart rates obtained by calculation may be taken as the final heart rate measurement.

It should further be appreciated that steps S641 to S644 are not essential. For example, the heart rate may be calculated directly from the pulse cycle obtained at step S330. This is acceptable in some low cost applications.

By employing (optionally) integrated reflection-type blood oxygen sensor and the improved differential threshold method, the pulse cycle detection device and method according to embodiments of the disclosure improve the timeliness and accuracy (the error is $\pm 2$bpm) of the pulse cycle (heart rate) detection in terms of both signal acquisition and signal processing, and may reduce the computational load, thereby providing the wearable pulse cycle (heart rate) detection device with a desirable option.

While several specific implementation details are contained in the above discussions, these should not be construed as limitations on the scope of any disclosure or of what may be claimed, but rather as descriptions of features that may be specific to particular embodiments of particular disclosures. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination (for example, as far as the function of pulse cycle detection is concerned, the display and/or the communication interface is not essential to the detection device 100), and the claimed combination may be directed to a sub-combination or variation of a sub-combination.

Similarly, while operations are depicted in the drawings in a particular order, this should not be understood as requiring that such operations are to be performed in the particular order shown or in a sequential order, or that all illustrated operations are to be performed to achieve desirable results (for example, in the case of calculating the pulse cycle from the pulse wave signal, the data acquisition step and the data preprocessing step, etc. are not essential).

The disclosure further provides a wearable electronic device comprising the pulse cycle detection device as described previously. The wearable electronic device may take the form of e.g. bracelet, wristband, neck-set, headphone, and thereby may be worn on a user. Thus, during the wearing time, the reflection-type blood oxygen sensor as described previously may acquire the pulse wave signal, and the pulse cycle detection device calculates the pulse or the heart rate from the pulse wave signal, and provides the wearable electronic device with corresponding detection information.

Various modifications, adaptations to the foregoing exemplary embodiments of this disclosure may become apparent to those skilled in the relevant arts in view of the foregoing description, when read in conjunction with the accompanying drawings. Any and all modifications will still fall within the scope of the non-limiting and exemplary embodiments of this disclosure. Furthermore, other embodiments of the disclosures set forth herein will come to mind to one skilled in the art to which these embodiments of the disclosure pertain having the benefit of the teachings presented in the foregoing descriptions and the associated drawings.

Therefore, it is to be understood that the embodiments of the disclosure are not to be limited to the specific embodiments disclosed and that modifications and other embodiments are intended to be included within the scope of the appended claims. Although specific terms are used herein, they are used in a generic and descriptive sense only and not for purposes of limitation.

## Claims

1. A pulse cycle detection device (100) comprising:

   a sensor (230) for sensing a pulse wave signal of a subject; and
   a processor for processing a digital pulse wave signal which is analog-to-digital converted from the pulse wave signal sensed by the sensor, to detect the pulse cycle of the subject,
   wherein the processor is configured to perform operations of:

   calculating a differential value of the digital pulse wave signal over time;
   determining that the pulse wave signal reaches a maximum point and recording a time value corresponding to the maximum point, whenever a transition from positive to negative occurs to the differential value;
   recognizing a last recorded maximum point as a peak point of the pulse wave signal, once the differential value is less than a dynamic threshold; and
   calculating a difference between time values corresponding to two successive peak points for deriving the pulse cycle.

2. The pulse cycle detection device as claimed in claim 1, wherein the processor is configured to calculate the difference between time values corresponding to two successive peak points for deriving the pulse cycle whenever the two successive peak points are recognized.

3. The pulse cycle detection device as claimed in claim 1, wherein the sensor is a reflection-type blood oxygen sensor comprising:

   a LED light source; and
   an integrated photodiode comprising multiple photodiode units integrated together for providing multi-channel sensing data.

4. The pulse cycle detection device as claimed in claim 3, wherein the LED light source is a green light LED.

5. The pulse cycle detection device as claimed in claim 3, wherein the multiple photodiode units are arranged side by side in a way in which their distances to the LED light source increase progressively, or arranged side by side in a way in which their distances to the LED light source are equal.

6. The pulse cycle detection device as claimed in claim 1, wherein the dynamic threshold is selected from the group consisting of 1/2 of a minimum differential value in an elapsed predetermined time interval, 1/2 of a minimum differential value in a last threshold update cycle, and 1/2 of a minimum differential value in a predetermined number of elapsed pulse cycles.

7. The pulse cycle detection device as claimed in claim 1, wherein the processor is configured to perform at least one of calculating the differential value of the digital pulse wave signal point by point and preprocessing before processing the digital pulse wave signal.

8. The pulse cycle detection device as claimed in claim 1, wherein the processor is further configured to calculate a heart rate directly from the pulse cycle.

9. The pulse cycle detection device as claimed in claim 1, wherein the processor is further configured to compare a difference in time between a currently measured pulse cycle and a last measured pulse cycle, and determine that the currently measured pulse cycle is valid if the difference in time is less than a predetermined threshold, and that the currently measured pulse cycle is invalid otherwise.

10. A pulse cycle detection method for processing a digital pulse wave signal to detect the pulse cycle of a subject, comprising:

calculating a differential value of the digital pulse wave signal over time;
determining that the pulse wave signal reaches a maximum point and recording a time value corresponding to the maximum point, whenever a transition from positive to negative occurs to the differential value;
recognizing a last recorded maximum point as a peak point of the pulse wave signal, once the differential value is less than a dynamic threshold; and
calculating a difference between time values corresponding to two successive peak points for deriving the pulse cycle.

11. The pulse cycle detection method as claimed in claim 10, wherein the calculating the difference between time values corresponding to two successive peak points comprises: calculating the difference between time values corresponding to two successive peak points whenever the two successive peak points are recognized.

12. The pulse cycle detection method as claimed in claim 10, wherein the dynamic threshold is selected from the group consisting of 1/2 of a minimum differential value in an elapsed predetermined time interval, 1/2 of a minimum differential value in a last threshold update cycle, and 1/2 of a minimum differential value in a predetermined number of elapsed pulse cycles.

13. The pulse cycle detection method as claimed in claim 10, wherein the differential value of the digital pulse wave signal is calculated point by point.

14. The pulse cycle detection method as claimed in claim 10, further comprising at least one of:

preprocessing the digital pulse wave signal before processing the digital pulse wave signal;
calculating a heart rate directly from the pulse cycle; and
comparing a difference in time between a currently measured pulse cycle and a last measured pulse cycle, and determining that the currently measured pulse cycle is valid if the difference in time is less than a predetermined threshold, and that the currently measured pulse cycle is invalid otherwise.

15. A wearable electronic device comprising a pulse cycle detection device as claimed in any of claims 1-9.


**Patentansprüche**

1. Vorrichtung zum Erkennen von Pulszyklen (100), umfassend:

einen Sensor (230) zum Erfassen eines Pulswellensignals eines Subjekts; und
einen Prozessor zum Verarbeiten eines digitalen Pulswellensignals, das aus dem vom Sensor erfassten Pulswellensignal analog zu digital umgewandelt wird, um den Pulszyklus des Subjekts zu erkennen,
wobei der Prozessor konfiguriert ist, die folgenden Operationen durchzuführen:

Berechnen eines Differenzwertes des digitalen Pulswellensignals im Laufe der Zeit;
Bestimmen, dass das Pulswellensignal einen Maximalpunkt erreicht, und Aufzeichnen eines dem Maximalpunkt entsprechenden Zeitwerts, wenn ein Übergang des Differenzwertes von positiv zu negativ erfolgt;
Anerkennen eines zuletzt aufgezeichneten Maximalpunktes als Spitzenpunkt des Pulswellensignals, sobald der Differenzwert kleiner als ein dynamischer Schwellenwert ist; und
Berechnen einer Differenz zwischen Zeitwerten, die zwei aufeinanderfolgenden Spitzenwerten entsprechen, zum Ableiten des Pulszyklus.

2. Vorrichtung zum Erkennen von Pulszyklen nach Anspruch 1, wobei der Prozessor so konfiguriert ist, dass er die Differenz zwischen Zeitwerten, die zwei aufeinanderfolgenden Spitzenpunkten entsprechen, zum Ableiten des Pulszyklus berechnet, wann immer die beiden aufeinanderfolgenden Spitzenpunkte anerkannt werden.

3. Vorrichtung zum Erkennen von Pulszyklen nach Anspruch 1, wobei der Sensor ein Blutsauerstoffsensor vom Reflexionstyp ist, umfassend:

   eine LED-Lichtquelle; und
   eine integrierte Fotodiode, die mehrere zusammen integrierte Fotodiodeneinheiten zum Bereitstellen von Mehrkanal-Erfassungsdaten umfasst.

4. Vorrichtung zum Erkennen von Pulszyklen nach Anspruch 3, wobei die LED-Lichtquelle eine Grünlicht-LED ist.

5. Vorrichtung zum Erkennen von Pulszyklen nach Anspruch 3, wobei die mehreren Fotodiodeneinheiten nebeneinander so angeordnet sind, dass ihre Abstände zur LED-Lichtquelle progressiv zunehmen, oder nebeneinander so angeordnet sind, dass ihre Abstände zur LED-Lichtquelle gleich sind.

6. Vorrichtung zum Erkennen von Pulszyklen nach Anspruch 1, wobei der dynamische Schwellenwert aus der Gruppe ausgewählt ist, die aus 1/2 eines minimalen Differenzwertes in einem verstrichenen vorbestimmten Zeitintervall, 1/2 eines minimalen Differenzwertes in einem letzten Schwellenwertaktualisierungszyklus und 1/2 eines minimalen Differenzwertes in einer vorbestimmten Anzahl von verstrichenen Pulszyklen besteht.

7. Vorrichtung zum Erkennen von Pulszyklen nach Anspruch 1, wobei der Prozessor so konfiguriert ist, dass er mindestens eines durchführt von einem Berechnen des Differenzwert des digitalen Pulswellensignals Punkt für Punkt oder einem Vorverarbeiten vor dem Verarbeiten des digitalen Pulswellensignals.

8. Vorrichtung zum Erkennen von Pulszyklen nach Anspruch 1, wobei der Prozessor ferner so konfiguriert ist, dass er eine Herzfrequenz direkt aus dem Pulszyklus berechnet.

9. Vorrichtung zum Erkennen von Pulszyklen nach Anspruch 1, wobei der Prozessor ferner so konfiguriert ist, dass er eine Zeitdifferenz zwischen einem aktuell gemessenen Pulszyklus und einem letzten gemessenen Pulszyklus vergleicht und bestimmt, dass der aktuell gemessene Pulszyklus gültig ist, wenn die Zeitdifferenz kleiner als ein vorbestimmter Schwellenwert ist, und dass der aktuell gemessene Pulszyklus andernfalls ungültig ist.

10. Verfahren zum Erkennen von Pulszyklen zum Verarbeiten eines digitalen Pulswellensignals zum Erkennen des Pulszyklus eines Subjekts, umfassend:

    Berechnen eines Differenzwertes des digitalen Pulswellensignals im Laufe der Zeit;
    Bestimmen, dass das Pulswellensignal einen Maximalpunkt erreicht, und Aufzeichnen eines dem Maximalpunkt entsprechenden Zeitwerts, wenn ein Übergang des Differenzwertes von positiv zu negativ erfolgt;
    Anerkennen eines zuletzt aufgezeichneten Maximalpunktes als Spitzenpunkt des Pulswellensignals, sobald der Differenzwert kleiner als ein dynamischer Schwellenwert ist; und
    Berechnen einer Differenz zwischen Zeitwerten, die zwei aufeinanderfolgenden Spitzenwerten entsprechen, zum Ableiten des Pulszyklus.

11. Verfahren zum Erkennen von Pulszyklen nach Anspruch 10, wobei das Berechnen der Differenz zwischen Zeitwerten, die zwei aufeinanderfolgenden Spitzenpunkten entsprechen, umfasst: Berechnen der Differenz zwischen Zeitwerten, die zwei aufeinanderfolgenden Spitzenpunkten entsprechen, wann immer die zwei aufeinanderfolgenden Spitzenpunkte anerkannt werden.

12. Verfahren zum Erkennen von Pulszyklen nach Anspruch 10, wobei der dynamische Schwellenwert aus der Gruppe ausgewählt ist, die aus 1/2 eines minimalen Differenzwertes in einem verstrichenen vorbestimmten Zeitintervall, 1/2 eines minimalen Differenzwertes in einem letzten Schwellenwertaktualisierungszyklus und 1/2 eines minimalen Differenzwertes in einer vorbestimmten Anzahl von verstrichenen Pulszyklen besteht.

13. Verfahren zum Erkennen von Pulszyklen nach Anspruch 10, wobei der Differenzwert des digitalen Pulswellensignals Punkt für Punkt berechnet wird.

**14.** Verfahren zum Erkennen von Pulszyklen nach Anspruch 10, ferner umfassend mindestens eines der Folgenden:

Vorverarbeiten des digitalen Pulswellensignals vor dem Verarbeiten des digitalen Pulswellensignals;
Berechnen einer Herzfrequenz direkt aus dem Pulszyklus; und
Vergleichen einer Zeitdifferenz zwischen einem aktuell gemessenen Pulszyklus und einem letzten gemessenen Pulszyklus, und Bestimmen, dass der aktuell gemessene Pulszyklus gültig ist, wenn die Zeitdifferenz kleiner als ein vorbestimmter Schwellenwert ist, und dass der aktuell gemessene Pulszyklus andernfalls ungültig ist.

**15.** Am Körper tragbare elektronische Vorrichtung, umfassend eine Vorrichtung zum Erkennen von Pulszyklen nach einem der Ansprüche 1 bis 9.

**Revendications**

**1.** Dispositif de détection de cycle d'impulsion (100), comprenant :

un capteur (230) pour détecter un signal d'onde d'impulsion d'un sujet ; et
un processeur pour traiter un signal d'onde d'impulsion numérique qui est converti d'analogique-à-numérique à partir du signal d'onde d'impulsion détecté par le capteur, de manière à détecter le cycle d'impulsion du sujet, dans lequel le processeur est conçu pour effectuer des opérations :

de calcul d'une valeur différentielle du signal d'onde d'impulsion numérique dans le temps ;
de détermination que le signal d'onde d'impulsion atteint un point maximum et d'enregistrement d'une valeur temporelle correspondant au point maximum, chaque fois que la valeur différentielle varie d'un nombre positif à un nombre négatif ;
d'identification du point maximum le plus récemment enregistré en tant que point de crête du signal d'onde d'impulsion, une fois que la valeur différentielle est inférieure à une valeur seuil dynamique ; et
de calcul d'une différence entre des valeurs temporelles correspondant à deux points de crête successifs pour dériver le cycle d'impulsion.

**2.** Dispositif de détection de cycle d'impulsion selon la revendication 1, dans lequel le processeur est conçu pour calculer la différence entre les valeurs temporelles correspondant à deux points de crête successifs pour dériver le cycle d'impulsion chaque fois que les deux points de crête successifs sont identifiés.

**3.** Dispositif de détection de cycle d'impulsion selon la revendication 1, dans lequel le capteur est un capteur d'oxygène sanguin du type à réflexion comprenant :

une source de lumière LED ; et
une photodiode intégrée comprenant plusieurs unités de photodiode intégrées ensemble pour fournir des données de détection multicanaux.

**4.** Dispositif de détection de cycle d'impulsion selon la revendication 3, dans lequel la source de lumière LED est une LED à lumière verte.

**5.** Dispositif de détection de cycle d'impulsion selon la revendication 3, dans lequel la pluralité d'unités de photodiode sont disposées côte à côte de manière à ce que leurs distances par rapport à la source de lumière LED augmentent progressivement, ou disposées côte à côte de manière à ce que leurs distances par rapport à la source de lumière LED sont égales.

**6.** Dispositif de détection de cycle d'impulsion selon la revendication 1, dans lequel la valeur seuil dynamique est choisie dans le groupe constitué d'1/2 d'une valeur différentielle minimale dans un intervalle de temps prédéterminé écoulé, d'1/2 d'une valeur différentielle minimale dans un dernier cycle de mise à jour de valeur seuil, et d'1/2 d'une valeur différentielle minimale dans un nombre prédéterminé de cycles d'impulsion écoulés.

**7.** Dispositif de détection de cycle d'impulsion selon la revendication 1, dans lequel le processeur est conçu pour effectuer au moins l' un du calcul de la valeur différentielle du signal d'onde d'impulsion numérique point par point et d'un prétraitement avant le traitement du signal d'onde d'impulsion numérique.

**8.** Dispositif de détection de cycle d'impulsion selon la revendication 1, dans lequel le processeur est en outre conçu pour calculer une fréquence cardiaque directement à partir du cycle d'impulsion.

**9.** Dispositif de détection de cycle d'impulsion selon la revendication 1, dans lequel le processeur est en outre conçu pour comparer une différence temporelle entre un cycle d'impulsion actuellement mesuré et un dernier cycle d'impulsion mesuré, et déterminer que le cycle d'impulsion actuellement mesuré est valide si la différence temporelle est inférieure à une valeur seuil prédéterminée, et que faute de quoi le cycle d'impulsion actuellement mesuré n'est pas valide.

**10.** Procédé de détection de cycle d'impulsion pour traiter un signal d'onde d'impulsion numérique de manière à détecter le cycle d'impulsion d'un sujet, comprenant :

le calcul d'une valeur différentielle du signal d'onde d'impulsion numérique dans le temps ;
le fait de déterminer que le signal d'onde d'impulsion atteint un point maximum et l'enregistrement d'une valeur temporelle correspondant au point maximum, chaque fois que la valeur différentielle varie d'un nombre positif à un nombre négatif ;
l'identification d'un point maximum le plus récemment enregistré en tant que point de crête du signal d'onde d'impulsion, une fois que la valeur différentielle est inférieure à une valeur seuil dynamique ; et
le calcul d'une différence entre des valeurs temporelles correspondant à deux points de crête successifs pour dériver le cycle d'impulsion.

**11.** Procédé de détection de cycle d'impulsion selon la revendication 10, dans lequel le calcul de la différence entre les valeurs temporelles correspondant à deux points de crête successifs comprend : le calcul de la différence entre les valeurs temporelles correspondant à deux points de crête successifs chaque fois que les deux points de crête successifs sont identifiés.

**12.** Procédé de détection de cycle d'impulsion selon la revendication 10, dans lequel la valeur seuil dynamique est choisie dans le groupe constitué d'1/2 d'une valeur différentielle minimale dans un intervalle de temps prédéterminé écoulé, d'1/2 d'une valeur différentielle minimale dans un dernier cycle de mise à jour de valeur seuil, et d'1/2 d'une valeur différentielle minimale dans un nombre prédéterminé de cycles d'impulsion écoulés.

**13.** Procédé de détection de cycle d'impulsion selon la revendication 10, dans lequel la valeur différentielle du signal d'onde d'impulsion numérique est calculée point par point.

**14.** Procédé de détection de cycle d'impulsion selon la revendication 10, comprenant en outre au moins l'un :

d'un prétraitement du signal d'onde d'impulsion numérique avant de traiter le signal d'onde d'impulsion numérique ;
du calcul d'une fréquence cardiaque directement à partir du cycle d'impulsion ; et
de la comparaison d'une différence temporelle entre un cycle d'impulsion actuellement mesuré et un dernier cycle d'impulsion mesuré, et le fait de déterminer que le cycle d'impulsion actuellement mesuré est valide si la différence temporelle est inférieure à une valeur seuil prédéterminée, et que faute de quoi le cycle d'impulsion actuellement mesuré n'est pas valide.

**15.** Dispositif électronique pouvant être porté, comprenant un dispositif de détection de cycle d'impulsion selon l'une quelconque des revendications 1 à 9.

Fig. 1

Fig. 2(a)

Fig. 2(b)

Fig. 2(c)

EP 3 311 737 B1

Fig. 3

Fig. 4

13

Set a dynamic differential threshold — S531

Record the coordinate of a maximum point of the pulse wave signal — S532

The differential value meet the dynamic threshold condition? — S533 No

Yes

Extract a peak point from recorded maximum points — S534

Calculate the difference in time of neighboring peak points — S535

Fig 5

Compare difference between neighboring pulse cycles — S641

Less than 100 milliseconds? — S642 No → Invalid data

Yes

Calculate the instantaneous heart rate — S643

Calculate the average heart rate — S644

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014243628 A1
- US 2003236647 A1
- US 2014114580 A1
- US 6575912 B1